(19) Europäisches Patentamt
European Patent Office
Office européen des brevets

(11) Publication number: **0 434 140 B1**

(12) **EUROPEAN PATENT SPECIFICATION**

(45) Date of publication of patent specification: **15.03.95**

(51) Int. Cl.[6]: **C07C 37/56**, C07C 37/055

(21) Application number: **90203305.9**

(22) Date of filing: **13.12.90**

(54) **Method for the preparation of a phenol.**

(30) Priority: **19.12.89 NL 8903098**

(43) Date of publication of application:
**26.06.91 Bulletin 91/26**

(45) Publication of the grant of the patent:
**15.03.95 Bulletin 95/11**

(84) Designated Contracting States:
**AT BE CH DE DK ES FR GB GR IT LI NL SE**

(56) References cited:
**GB-A- 824 631**
**GB-A- 2 001 317**
**US-A- 2 766 294**

(73) Proprietor: **DSM N.V.**
**Het Overloon 1**
**NL-6411 TE Heerlen (NL)**

(72) Inventor: **Buijs, Wim**
**Wolfhagen 145**
**NL-6365 BM Schinnen (NL)**
Inventor: **Frijns, Leon Hubertus Barbara**
**Cremerstraat 8**
**NL-6301 GE Valkenburg (NL)**
Inventor: **Offermanns, Matthias Robert Jozef**
**Kopperslagerstraat 20**
**NL-6151 DK Munstergeleen (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 434 140 B1**

Rank Xerox (UK) Business Services
(3.10/3.09/3.3.3)

## Description

The invention relates to a method for the preparation of a phenol by oxidative decarboxylation in the liquid phase of the corresponding aryl carboxylic acid.

The preparation of a phenol by an oxidative decarboxylation has been known for a long time. NL-B-90.684 already disclosed such a process, with oxidation, decarboxylation and hydrolysis all being carried out in one single process step, at a temperature of at least 200 °C and preferably 230-250 °C.

This so-called Dow phenol process has been the subject of a number of patent publications, which aimed to eliminate the major drawback of the above process, i.e. the formation of quite some byproducts, mainly in the form of tar.

GB-A-1290626, for instance, discloses a two-step process for the preparation of a phenol from a benzene monocarboxylic acid. First, oxidation and decarboxylation are simultaneously effected at a temperature of 230-240 °C. The corresponding phenyl benzoate obtained is subsequently hydrolysed in the presence of oxygen at a temperature of about 200 °C.

GB-A-2001317 describes a method for the preparation of a phenol on the basis of a three-step process. According to GB-A-2001317, first an oxidation is carried out at a temperature of preferably 120-170 °C. Before proceeding to the decarboxylation step the water formed during this oxidation step has to be eliminated. In order to prevent tar formation, it is necessary to avoid the simultaneous presence of phenylbenzoate, water and an oxidizing agent.

In a second step a decarbonylation in the absence of oxygen and water is carried out, at a temperature of, preferably, lower than 220 °C, and the third step comprises hydrolysis of the aryl benzoate obtained, carried out in the absence of oxygen and preferably at a temperature of about 220 °C. According to the applicant of the above-mentioned patent, the presence of water in the decarboxylation is to be avoided, which is achieved by addition of a dehydrating agent, by an azeotropic distillation with an extra hydrocarbon added, or by stripping with a dry, inert gas.

However, all the processes referred to have not been able to realize phenol formation at a high yield in an economically sound way.

According to GB-A-2001317, for instance, a very large amount of catalyst is used, such that it takes the form of a separate, solid phase, rather than a homogeneous one, with all consequences thereof (including troublesome separation from the reaction mixture of the metal oxides formed). In addition, the methods employed to avoid the presence of water in the decarboxylation are rather expensive and laborious; the high yields cited cannot be reproduced.

GB-A-824631 describes a gas phase process for the preparation of phenol from benzoic acid, wherein copperoxide is used as the oxidizing agent. According to GB-A-824631 the oxidation, decarboxylation and hydrolysis of benzoic acid are performed in one reaction step, in the absence of oxygen. The reaction mixture is heated in two stages, respectively to 200-275 °C, followed by heating to 300-400 °C. Phenol is displaced from the reduced copper oxide by purging with an inert gas or steam or a mixture of both. The process described in GB-A-824631 however has the disadvantages that metallic copper is formed in the course of the reaction which clogs the reactor, whereby the selectivity to phenol is low and tar is formed as a byproduct.

The method according to the invention provides a process for the preparation of a phenol from the corresponding aryl carboxylic acid in which the drawbacks encountered in the above-mentioned methods are eliminated. Thus, a process is obtained in which high preparation selectivities are combined with an economically attractive embodiment. The present invention relates to a method for the preparation of a phenol by oxidative decarboxylation in the liquid phase of the corresponding aryl carboxylic acid in the presence of a dissolved Cu-containing catalyst, the phenol being obtained through a hydrolysis step wherein the folowing process steps are carried out:

a) catalyst oxidation at a temperature of 120-190 °C;

b) reaction of the aryl carboxylic acid in question with the oxidised catalyst of step a), carried out in the absence of oxygen and with the aid of water, at a temperature of 225-270 °C;

c) separation of the phenol thus obtained and recycling of the (reduced) catalyst to step a).

By performing the process in this way, a two-step process is obtained: first an oxidation, then combined (intermediate) formation of the arylarylcarboxylate (the reduction) and hydrolysis of this ester to the phenol. Combining the formation of the ester with the hydrolysis to the phenol has a strong positive effect on the overall yield. Also the number of separate process steps is reduced in comparison with the process described in GB-A-2001317. Of course these process steps (reduction and hydrolysis) according to the invention, under the conditions as outlined, can also be effected separately (which may make it possible to apply some variations in the reaction conditions for the reduction compared with those for hydrolysis), but

the possibility to use one single reactor for the combined reactions is of greater advantage.

Here and below, aryl carboxylic acid is understood to be a compound having the following structure:

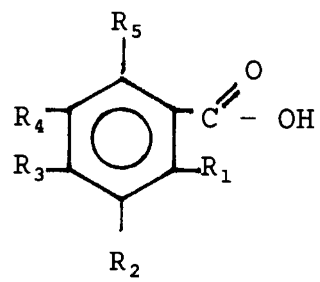

(I)

where $R_1$ through $R_5$ may be hydrogen (on the proviso that at least R1 or $R_5$ is hydrogen) or the following groups, substituted or not, which have a so-called Hammett constant of between -1 and +2. A description of this Hammett or $\sigma$ value, which represents a measure of the influence of the group on the reactivity of the aryl carboxylic acid, can be found in J. March, Advanced Organic Chemistry 1989, pages 242-250; reference is made in particular to table 4 on page 244. The groups that can be used therefore are: $C_1$-$C_6$ alkyl, cycloalkyl, aryl, arylalkyl, amino, halogen, nitro.

Salts, esters and anhydrides of (I) are also suitable, while groups may also be connected through a ring system, as is the case for instance in naphthalene carboxylic acid. Likewise, polyaryl carboxylic acids, such as trimellitic acid and pyromellitic acid, can be used as starting materials. Mixtures of the aryl carboxylic acids described in the above also are eligible for application of the method according to the invention.

The invention relates particularly to a method for the conversion of unsubstituted benzoic acid ($R_1$ through $R_5$ = hydrogen) into the corresponding unsubstituted phenol.

The oxidation of the Cu-containing catalyst, with conversion of Cu(I) arylcarboxylate into Cu(II) arylcarboxylate, is a first reaction step in the process. It brings about an increase in the degree of oxidation of the copper (from 1+ to 2+), while an extra arylcarboxylate part is incorporated in the Cu-containing catalyst. The process conditions for this need to be chosen such that on the one hand the reaction can be carried out in the liquid phase (i.e. above the melting point of the aryl carboxylic acid that is to be converted), but on the other at such a temperature that the following steps (viz. reduction and hydrolysis) cannot yet occur. This latter requirement renders it necessary to avoid temperatures above 190 °C, and certainly those above 210 °C, in the first process step. To even more avoid the following steps and to suppress side reactions, the catalyst oxidation is to be carried out in the (virtual) absence of the phenol to be formed. The presence of the phenol (for instance due to recycle flows) in the oxidation may give rise to the formation of tarry byproducts that adversely affect the overall selectivity.

Oxidation of Cu(I) to Cu(II) proceeds well particularly if carried out using an oxygen-containing gas. Air, whether or not enriched with oxygen, is very suitable for this. Such a gas can be passed through the Cu-containing liquid, for instance in a bubble column. The pressure applied is not critical, but in general an elevated pressure will be chosen so as to accelerate the oxidation process. Pressures of 0.1-2.5 MPa therefore are suitable.

An alternative and very suitable method for bringing about the conversion of Cu(I) into Cu(II) is to use the effect of an electrochemical potential, in which case the abstraction of an electron enables the desired copper conversion to take place.

The applicant has found that for the conversion of Cu(I) into Cu(II) using an oxygen-containing gas (or other means by which Cu oxidation can be effected via an oxygen group) preference is to be given to the use of a deficiency of oxygen relative to the amount of Cu(I) in the catalyst. This way it is ensured that not all of the Cu is in the Cu(II) form. It is particularly desirable to oxidise 30-95% of the Cu(I) to Cu(II). For electrochemical conversion of Cu(I) to Cu(II) such a measure was found to be unnecessary.

The amount of Cu-containing catalyst is to be chosen so that on the one hand a good activity is obtained, but on the other hand it should not be so large as to give rise to the presence of a separate, solid catalyst phase throughout the process. In the homogeneous process the catalyst is dissolved in the reaction mixture, though some excess in the oxidation step (where the temperature is much lower than in the following step) is allowable. The copper concentration (as a metal) in the oxidation step therefore is 0.5-15 wt. %, more preferably 1-10 wt. %; and ideally the process is carried out using a copper concentration between 1.5 and 8 wt. % (all figures relating to the reaction mixture in the oxidation step).

There may be advantage in using a catalyst containing a co-catalyst besides copper. This co-catalyst can be chosen in particular from groups V, VI, VII and VIII as well as from the group of the lanthanides and actinides of the Periodic System of the Elements. These components affect the oxidative capacity of the Cu in the catalyst. Furthermore, promoters may be used, suitable substances being in particular (earth) alkaline metals, such as Mg or Li.

It is especially preferred to oxidise the Cu-containing catalyst, according to any of the processes described in the above, at a temperature of 150-180 °C.

The second step in the method according to the invention comprises a combination of a reduction (yielding the aryl aryl carboxylate) and hydrolysis to the phenol, with carbondioxide ($CO_2$) being separated. Here and hereafter aryl aryl carboxylate is understood to be a compound having the following structure

$$\varnothing_2 - \underset{\underset{\displaystyle O}{\parallel}}{C} - O - \varnothing_1 \qquad (II)$$

where $\varnothing_1$ and $\varnothing_2$ is an aryl group, substituted or unsubstituted, as found in structure (I). When performing the reduction and hydrolysis step separately (as in GB-A-2001317) first o-, m-and p-arylcarboxy-aryl carboxylic acids are formed, which are the products of two aryl carboxylic acids coupled to one another. The o- and p-products are converted into the corresponding phenol by the subsequent hydrolysis. The m-products, which are formed in an amount of 25-50% of the products, cannot, under the indicated process conditions, be converted to the corresponding phenols.

It seems probable that the choice of process conditions in the second step according to the invention results in a strong suppression of the formation of the m- and p-products. The o-product is immediately, under the process conditions of the invention, converted into the corresponding phenol. If no water is applied in said reduction, undesirable byproducts are formed which cannot be converted into the corresponding phenol.

In contrast to what is stated in GB-A-2001317, it thus has proved essential in the second step of the method according to the invention for water to be present in the reduction step. This makes it possible to combine this reduction into one process step with the subsequent hydrolysis step, in which water must necessarily be present. This can be done particularly well because the temperature at which this reduction is carried out is higher than that preferred in GB-A-2001317 (which cites a temperature ≦ 220 °C; according to the invention 225-270 °C). It has been found that the use of a temperature of 225-270 °C, and preferably a temperature of 230-250 °C, gives rise to an improvement of the overall selectivity.

According to the invention the hydrolysis step yielding the phenol should also take place at a temperature of 225-270 °C and preferably of 235-250 °C. This temperature is significantly higher than that applied for hydrolysis in GB-A-1290626, viz. about 200 °C.

Both the reduction and the hydrolysis take place in the absence of oxygen, this in contrast to the process disclosed in GB-A-1290626. Oxygen being absent, re-oxidation of the Cu-containing catalyst in this process step is avoided, so that there can be no, disadvantageous, reactions between the phenol, or intermediates thereof, and such an oxidised catalyst, which would give rise to the formation of selectivity-depressing byproducts (such as tar).

Before feeding the reaction mixture from the first step (the oxidation step) to the second process step (the reduction and hydrolysis step) it is advantageous to free the reaction mixture of part of the aryl carboxylic acid contained in it. The degree to which this is possible is determined by the solubility of the copper-containing catalyst in the reaction mixture under the process conditions of the second step. Partial evaporation (for instance by distillation) is one of the methods by which an amount of aryl carboxylic acid can be removed; this aryl carboxylic acid can, depending on its quality, be passed to the upgrading section (more on which later) or fed direct to the oxidation step.

It is advantageous to use such an amount of water in the second step of the process according to the invention as to achieve virtual equimolarity relative to the amount of aryl aryl carboxylate. As a result, the reaction product obtained upon completion of the hydrolysis is almost free of water, which is of advantage in further upgrading to pure phenol of the reaction product thus obtained.

The pressure under which the second process step is performed is not critical, but the advantage of raising the pressure to above atmospheric level is that it has a favourable effect on the reaction kinetics and that the volatility of the reaction product is reduced. The pressure to be applied will generally be between 0.1 and 2.5 MPa; higher pressures, though allowable, do not yield substantial improvements of the process.

After the second step the reaction mixture is subjected to an upgrading operation to separate and recover the phenol obtained. This can be done in ways known by themselves, for instance by distillation.

Certainly when hardly any water is left in the reaction mixture (in contrast to the process described in NL-B-90.684, which is also referred to as the 'wet route'), there is no longer any need to use an auxiliary material in the distillation (such as toluene to break the phenol-water azeotrope). The bottom flow of the distillation, containing non-converted aryl carboxylic acid and the Cu-containing catalyst, can be recycled to the oxidation step, optionally after a purification step.

The process according to the invention is particularly suitable for the preparation of unsubstituted phenol from unsubstituted benzoic acid. This phenol can be used, for instance, as a basic material for both phenol-formaldehyde resins and the preparation of caprolactam, the nylon-6 feedstock.

The invention will be elucidated in the following examples. However, these examples should not be construed as limiting the invention.

The following examples have all been conducted in an oil-heated, double-walled reactor having an effective volume of 0.5 l. This reactor was provided with a stirrer, gas inlet, steam inlet, gas outlet and a distillation set-up. Use was made of a gas cylinder filled with a gas of a calibrated composition (nitrogen with 4.8 vol. % oxygen), to feed gas to the reactor. After cooling of the condensible products, the off-gas was analysed for oxygen and carbon dioxide. In all experiments, which were performed batchwise, use was made of 350 g of reactor mass, consisting of the desired amounts of catalyst, co-catalyst and promoter, with aryl carboxylic acid added up to the total weight. During the experiment no make-up supplies were made to the reactor mass. The catalyst, co-catalyst and promoter were supplied as the metal oxide or as the metal aryl carboxylate, with identical results in both cases.
Upon completion of the reaction, the reaction product obtained and the reactor mass were subjected to HPLC (High Pressure Liquid Chromatography) analysis. The results presented in Table I were obtained as follows:

a) the phenol yield ($\Sigma$ F) is expressed as the number of moles obtained of phenol + aryl aryl carboxylate + p-arylcarboxy arylcarboxylic acid + p-hydroxyaryl carboxylic acid;
b) the yield of m-product ($\Sigma$ m-product) is expressed as the number of moles obtained of m-arylcarboxy arylcarboxylic acid + m-hydroxyaryl carboxylic acid;
c) the yield of total products ($\Sigma$ products) is expressed as $\Sigma$F + $\Sigma$ m-products;
d) the selectivity towards phenol (SF) is expressed as

$$\frac{\Sigma\ F}{\Sigma\ \text{products}} \times 100\%$$

In none of the experiments was formation of tar-like products observed, while in recycle examples V and VI no catalyst deactivation was observed. For all examples the mass, aromatic ring, $CO_2$ and $O_2$ balances had a value between 99% and 101%.

<u>Experiment I</u>

The gas was passed through a reactor mass on the basis of 1.0 wt. % of Cu (I) in benzoic acid at a flow rate of 30 Nl/h during 18 minutes, the temperature being 180 °C and the pressure 0.1 MPa. From the gas balance and the oxygen absorption derived from this balance, the conversion of Cu (I) to Cu (II) was found to be 95%.

<u>Example II</u>

Experiment I was repeated, now 3.0 wt. % of Cu (II) being used. After the oxidation a combined reduction and hydrolysis (second step) was performed. To this end 30 g/h of water was supplied; the reaction was carried out at a temperature of 230 °C and a pressure of 0.1 MPa.

<u>Example III</u>

In this example the conditions of Example II were maintained except that the temperature of the second step now was 240 °C.

Example IV

In this example the reactor mass contained, besides benzoic acid, 3.0 wt. % of Cu (II) and 3.5 wt. % of Mg (II), the further process conditions being as in Example II.

Example V

Example II was repeated, and upon completion fresh benzoic acid was added to the reactor mass obtained, which was then subjected to an oxidation step in which 30 Nl/h of the gas was passed through during 18 minutes, at a temperature of 170 °C, following which the second step was performed again. This was repeated 3 times.

Example VI

Example IV was repeated, and upon completion fresh benzoic acid was added to the reactor mass obtained, which was then subjected during 18 minutes to an oxidation step at a temperature of 180 °C. 30 Nl/h of air was passed through. After this, the second step was performed again. This was repeated 5 times.

Example VII

The reactor mass of Example V was subjected to a supplementary hydrolysis reaction during 20 hours by feeding 30 g/h water at 230 °C and a pressure of 0.1 MPa to check if further conversion of the m-products still present into the phenol is possible.
The results of the experiments and the examples are summarised in the following Table I.

Table I

Results

| No. | time h | temp. °C | pressure $10^5$ Pa | Σ F mmol | Σm-product mmol | SF % |
|---|---|---|---|---|---|---|
| I | 0,3 | 180 | 1,0 | 0 | 0 | 0 |
| II | 4,0 | 230 | 1,0 | 73 | 9 | 89,0 |
| III | 4,0 | 240 | 1,0 | 75 | 7 | 91,5 |
| IV | 4,0 | 230 | 1,0 | 83 | ≤1 | ≥99 |
| V | 0,3 | 170 | 1,0 | 208 | 15 | 93.3 |
| | 4,0 | 230 | | | | |
| VI | 0,3 | 180 | 1,0 | 408 | ≤1 | ≥99 |
| | 4,0 | 230 | | | | |
| VII | 20 | 230 | 1,0 | 208 | 15 | 93.3 |

Comparative experiment A

Example I of NL-A-78.07199 was repeated at a temperature of 218 °C and during an experimental period of 1 hour, the Cu (II) concentration, for obtaining a homogeneous catalyst application, being 4.0 mol %.

Comparative experiment B

The preceding experiment, A, was repeated, but now during a period of 2 hours.

Comparative experiment C

Comparative experiment B was repeated, use being made also of 3.5 wt. % Mg (II).

Comparative experiment D

An attempt was made to repeat Example III of NL-A-78.07199, but under the conditions as specified there it proved impossible, at atmospheric pressure, to obtain a temperature above 150 °C, so that no phenol formation reaction occurred. By adjusting the xylene content to 10 wt. % relative to the reactor mass the temperature could be raised to 215 °C. The further conditions of this comparative experiment are the same as those of comparative experiment A.
In the following the results are presented of examples using substituted benzoic acids. In all cases both reaction steps were analogous to those of Example II. The Cu (II) concentration was 4.0 mol %.

Examples VIII and XII

Analogous to Example II, in the examples m-methyl benzoic acid and m-dimethyl amino benzoic acid, respectively, were used as starting material. Also, use was made of 3.5 wt. % Mg (II).

Examples IX-XI

In these examples p-t-butyl benzoic acid, m-Cl-benzoic acid and m-$NO_2$-benzoic acid, respectively, were used as starting materials, the further process conditions being analogous to those of Example II.
When Example IX was repeated, the presence of 3.5 wt. % Mg (II) appeared to lead to a lowering of the reaction velocity, but not to a different selectivity.
The results of Comparative experiments A-D and Examples VIII-XII are presented in Table II.

Table II

| Results | | | | | | |
|---|---|---|---|---|---|---|
| No. | time h | temp. °C | pressure $10^5$ Pa | ΣF mmol | Σm-product mmol | SF % |
| A | 1.0 | 218 | 1.0 | 46 | 35 | 56,8 |
| B | 2.0 | 218 | 1.0 | 47 | 37 | 56,0 |
| C | 2.0 | 218 | 1.0 | 100 | 34 | 74,6 |
| D | 1.0 | 218 | 1.0 | 100 | 102 | 49,5 |
| VIII | 1.0 | 230 | 1.0 | 132 | <1 | >99 |
| IX | 1.0 | 230 | 1.0 | 126 | <1 | >99 |
| X | 1.0 | 230 | 1.0 | 51 | <1 | >99 |
| XI | 1.0 | 230 | 1.0 | 106 | <1 | >99 |
| XII | 1.0 | 250 | 1.0 | 23 | 2 | 92,0 |

From this table it can be concluded that:

*) under the process conditions of GB-A-2001317 a large amount of m-product is formed, so that the selectivity towards the desired product is low;

*) substituted benzoic acids, too, can according to the invention be converted into the corresponding phenols at high selectivities.

**Claims**

**1.** Method for the preparation of a phenol by oxidative decarboxylation in the liquid phase of the corresponding aryl carboxylic acid in the presence of a dissolved Cu-containing catalyst, the phenol being obtained through a hydrolysis step, wherein the following process steps are carried out:

a) catalyst oxidation at a temperature of 120-190 °C;

b) reaction of the aryl carboxylic acid in question with the oxidised catalyst of step a), carried out in the absence of oxygen and with the aid of water, at a temperature of 225-270 °C;

c) separation of the phenol thus obtained and recycling of the (reduced) catalyst to step a).

**2.** Method according to claim 1, characterised in that an oxygen-containing gas is used for the catalyst oxidation.

**3.** Method according to claim 2, characterised in that the oxidation is carried out using a deficiency of oxygen relative to the amount of Cu in the catalyst.

**4.** Method according to claim 1, characterised in that for the catalyst oxidation use is made of the effect of an electrochemical potential.

**5.** Method according to any one of claims 1-4, characterised in that the Cu-containing catalyst also contains a co-catalyst.

**6.** Method according to any one of claims 1-5, characterised in that step a) is carried out at a temperature of 150-180 °C.

**7.** Method according to any one of claims 1-6, characterised in that the concentration of copper (as a metal and relative to the reaction mixture) in the oxidation step amounts to 1-10 wt. %.

**8.** Method according to any one of claims 1-7, characterised in that step b) is carried out using an amount of water that is virtually equimolar relative to the amount of aryl arylcarboxylate.

**9.** Method according to any one of claims 1-8, characterised in that the reaction mixture of step a) is partly freed of the aryl carboxylic acid before being supplied to step b).

**10.** Method according to any one of claims 1-9, characterised in that step b) is carried out at a temperature of 230-250 °C.

**11.** Method according to any one of claims 1-10, characterised in that unsubstituted benzoic acid is used as aryl carboxylic acid.

**Patentansprüche**

**1.** Verfahren zur Herstellung eines Phenols durch oxidative Decarboxylierung der korrespondierenden Arylcarbonsäure in flüssiger Phase in Gegenwart eines gelösten, Cu-enthaltenden Katalysators, wobei das Phenol durch einen Hydrolyseschritt erhalten wird, worin die folgenden Verfahrensschritte ausgeführt werden:

a) Katalysatoroxidation bei einer Temperatur von 120-190 °C;

b) Umsetzung der in Frage stehenden Arylcarbonsäure mit dem oxidierten Katalysator von Schritt a), welche bei Abwesenheit von Sauerstoff und mit Hilfe von Wasser bei einer Temperatur von 225-270 °C ausgeführt wird;

c) Abtrennung des auf diese Weise erhaltenen Phenols und Rückführen des (reduzierten) Katalysators zu Schritt a).

**2.** Verfahren nach Anspruch 1, dadurch gekennzeichnet, daß ein Sauerstoff-enthaltendes Gas für die Katalysatoroxidation verwendet wird.

**3.** Verfahren nach Anspruch 2, dadurch gekennzeichnet, daß die Oxidation unter Anwendung eines Sauerstoffmangels relativ zur Menge an Cu im Katalysator ausgeführt wird.

**4.** Verfahren nach Anspruch 1, dadurch gekennzeichnet, daß für die Katalysatoroxidation die Wirkung eines elektrochemischen Potentials ausgenützt wird.

5. Verfahren nach einem der Ansprüche 1-4, dadurch gekennzeichnet, daß der Cu-enthaltende Katalysator auch einen Co-Katalysator enthält.

6. Verfahren nach einem der Ansprüche 1-5, dadurch gekennzeichnet, daß der Schritt a) bei einer Temperatur von 150-180 ° C ausgeführt wird.

7. Verfahren nach einem der Ansprüche 1-6, dadurch gekennzeichnet, daß die Konzentration an Kupfer (als Metall und relativ zum Reaktionsgemisch) im Oxidationsschritt 1-10 Gew.-% ausmacht.

8. Verfahren nach einem der Ansprüche 1-7, dadurch gekennzeichnet, daß der Schritt b) unter Anwendung einer Wassermenge ausgeführt wird, welche relativ zur Menge an Arylarylcarboxylat praktisch äquimolar ist.

9. Verfahren nach einem der Ansprüche 1-8, dadurch gekennzeichnet, daß das Reaktionsgemisch von Schritt a), bevor es dem Schritt b) zugeführt wird, von der Arylcarbonsäure teilweise befreit wird.

10. Verfahren nach einem der Ansprüche 1-9, dadurch gekennzeichnet, daß der Schritt b) bei einer Temperatur von 230-250 ° C ausgeführt wird.

11. Verfahren nach einem der Ansprüche 1-10, dadurch gekennzeichnet, daß unsubstituierte Benzoesäure als Arylcarbonsäure verwendet wird.

**Revendications**

1. Procédé de préparation d'un phénol par décarboxylation oxydante en phase liquide de l'acide arylcarboxylique correspondant en présence d'un catalyseur dissous contenant du Cu, le phénol étant obtenu par un stade d'hydrolyse, dans lequel on effectue les stades opératoires suivants :
   a) l 'oxydation du catalyseur à une température de 120 à 190 ° C ;
   b) la réaction de l' acide arylcarboxylique en question avec le catalyseur oxydé du stade a) , qu'on effectue en l'absence d'oxygène et à l'aide d'eau à une température de 225 à 270 ° C ;
   c) la séparation du phénol ainsi obtenu et recyclage du catalyseur (réduit) au stade a).

2. Procédé selon la revendication 1, caractérisé en ce qu'on utilise un gaz contenant de l'oxygène pour l'oxydation du catalyseur.

3. Procédé selon la revendication 2, caractérisé en ce qu'on effectue l'oxydation en utilisant une déficience en oxygène par rapport à la quantité de Cu dans le catalyseur.

4. Procédé selon la revendication 1, caractérisé en ce que pour l'oxydation du catalyseur, on utilise l'effet d'un potentiel électrochimique.

5. Procédé selon l'une quelconque des revendications 1 à 4, caractérisé en ce que le catalyseur contenant du Cu renferme également un co-catalyseur.

6. Procédé selon l'une quelconque des revendications 1 à 5, caractérisé en ce qu'on effectue le stade a) à une température de 150 à 180 ° C.

7. Procédé selon l'une quelconque des revendications 1 à 6 caractérisé en ce que la concentration en cuivre (en métal et par rapport au mélange de réaction) au stade d'oxydation est de 1 à 10% en poids.

8. Procédé selon l'une quelconque des revendications 1 à 7, caractérisé en ce qu'on effectue le stade b) en utilisant de l'eau en une quantité virtuellement équimolaire à la quantité d'arylcarboxylate d'aryle.

9. Procédé selon l'une quelconque des revendications 1 à 8, caractérisé en ce qu'on débarrasse partiellement le mélange de réaction du stade a) d'acide arylcarboxylique avant de l'admettre au stade b).

**10.** Procédé selon l'une quelconque des revendications 1 à 9, caractérisé en ce qu'on effectue le stade b) à une température de 230 à 250 °C.

**11.** Procédé selon l'une quelconque des revendications 1 à 10, caractérisé en ce qu'on utilise l'acide benzoîque non substitué à titre d'acide arylcarboxylique.